# EUROPEAN PATENT SPECIFICATION

(11) **EP 2 276 347 B1**
(45) Date of publication and mention of the grant of the patent: **25.05.2016**
(21) Application number: 09726974.0
(22) Date of filing: 03.04.2009
(51) Int. Cl.: A01N 43/66, A61K 9/16, A61K 31/53, A61K 9/08, A61K 9/10, A61K 9/14

(54) **TOLTRAZURIL WITH IMPROVED DISSOLUTION PROPERTIES**
TOLTRAZURIL MIT VERBESSERTEN AUFLÖSUNGSEIGENSCHAFTEN
TOLTRAZURIL DOTÉ DE PROPRIÉTÉS DE DISSOLUTION AMÉLIORÉES

(30) Priority: 03.04.2008 SI 200800078
(43) Date of publication of application: 26.01.2011
(73) Proprietor: KRKA, tovarna zdravil, d.d., Novo mesto, 8501 Novo mesto (SI)
(72) Inventor: TIHI, Jaroslav, 8000 Novo mesto (SI); BENKIC, Primoz, 1000 Ljubljana (SI); TOPORISIC, Rebeka, 1000 Ljubljana (SI); GOJAK, Urska, 8000 Novo mesto (SI); KLAVDIJA, Meznar, 8000 Novo mesto (SI); TROST, Sabina, 1330 Kocevje (SI)
(74) Representative: Uexküll & Stolberg
(86) International application number: PCT/EP2009/054012
(87) International publication number: WO 2009/121957

(56) References cited:
- EP-A- 0 130 160
- WO-A-01/26660
- WO-A-02/14288
- US-A- 4 606 939
- US-A1- 2006 280 786
- US-A1- 2007 104 860
- US-A1- 2007 254 008
- US-B1- 6 436 935
- US-B1- 6 465 460

## Description

### FIELD OF THE INVENTION

The present invention relates to toltrazuril with improved dissolution properties and to methods for its preparation.

### BACKGROUND OF THE INVENTION

Toltrazuril is a triazinetrione derivative used as anticoccidial agent. It is widely used in chicken, turkeys, pigs and cattle for the prevention and treatment of coccidiosis, by administration via drinking water. It is currently available on the marked as Baycox® 2.5% solution and 5% suspension.

Toltrazuril, methods for its preparation and compositions are disclosed in various patents such as GB 1 461 375, US 4,219,552, US 5,219,853, EP 0 201 030, EP 0 879 057, DE 35 16 631, DE 42 39 000, CN1896068, CN101108831, CN1927846, CN101265236 and WO 2008/145281.

WO 02/14288 discloses triazinetrione sulfones which are said to be useful for combating coccidiosis. The triazinetrione sulfones are preferably used as micronised particles in suspension.

US 6,436,935 concerns orally administrable pastes of toltrazuril sulfone. The active compound has a particle size of 1 to 10 µm.

US 6,465,460 discloses compositions comprising an anti-protozoal drug such as the sodium salt of toltrazuril which are adapted for parenteral administration.

WO 01/26660 discloses compositions for the treatment of protozoan infections comprising an anticoccidial agent such as toltrazuril dissolved in a suitable solvent such as DMSO or DMA. US 4,606,939 discloses a process for forming small particles of a weakly acidic organic compound whose solubility is water is greater at a first pH than at a second pH comprising (a) dissolving said compound in water in the presence of a base together with an anionic surfactant and an amphoteric surfactant, and (b) titrating the solution with a titrant effective to reduce the pH to cause formation of a coacervate of the anionic and amphoteric surfactants, and precipitation of the compound as small particles.

US 2006/0280786 concerns a pharmaceutical combination for minimizing pharmacokinetic drug-drug-interactions comprising a first pharmaceutical compound having a particular pharmacokinetic profile and a second pharmaceutical component having an altered pharmacokinetic profile different from the unaltered pharmacokinetic profile of the second pharmaceutical component, which would interfere with the pharmacokinetic profile of the first pharmaceutical component. Due to its altered pharmacokinetic profile, the second pharmaceutical component is said not to substantially affect the pharmacokinetic profile of the first pharmaceutical component.

US 2007/0254008 discloses a pharmaceutical formulation comprising a plurality of antimicrobial particles comprised of an antimicrobial drug and a biocompatible polymer; and a pharmaceutically acceptable carrier having the antimicrobial particles dispersed therein.

US 2007/0104860 concerns an all-dry encapsulation method that is said to enable well-defined polymers to be applied around particles of sizes down to the nanoscale. The particles to be coated are contacted with a reactive mixture of a gaseous monomer and a gaseous initiator.

Toltrazuril prepared according to methods disclosed in prior art has particles which are not optimal for pharmaceutical compositions, especially suspensions because their dissolution rate is too slow. Obvious solutions for improving dissolution by increasing particle specific surface area, such as milling, have been tried as well. However, particles were still not appropriate. Therefore there exists a constant need for increasing specific surface area of toltrazuril to improve its dissolution profile.

### BRIEF DESCRIPTION OF THE DRAWINGS

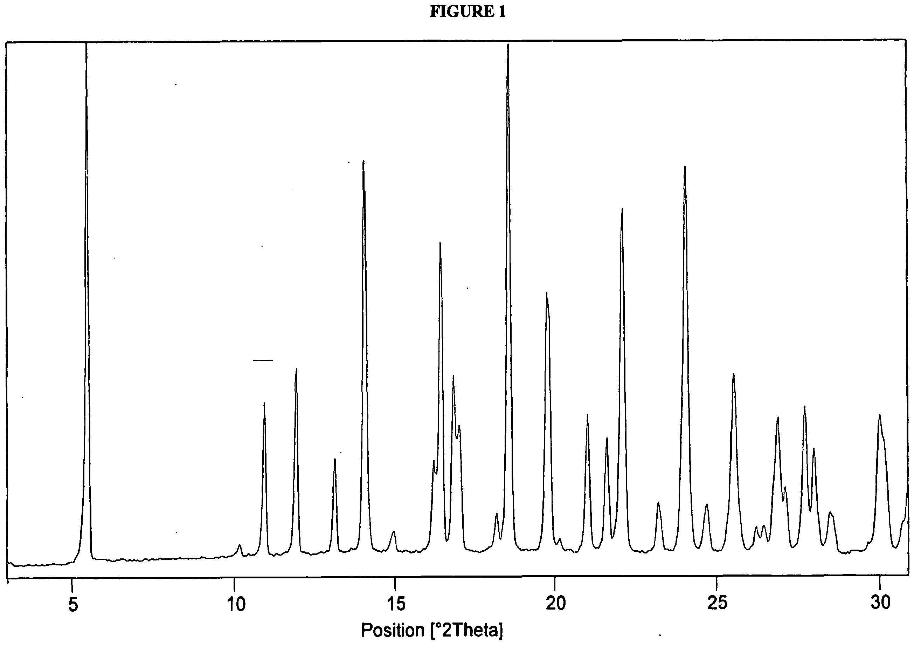

Figure 1 is an XRPD pattern of toltrazuril prepared according to example 1

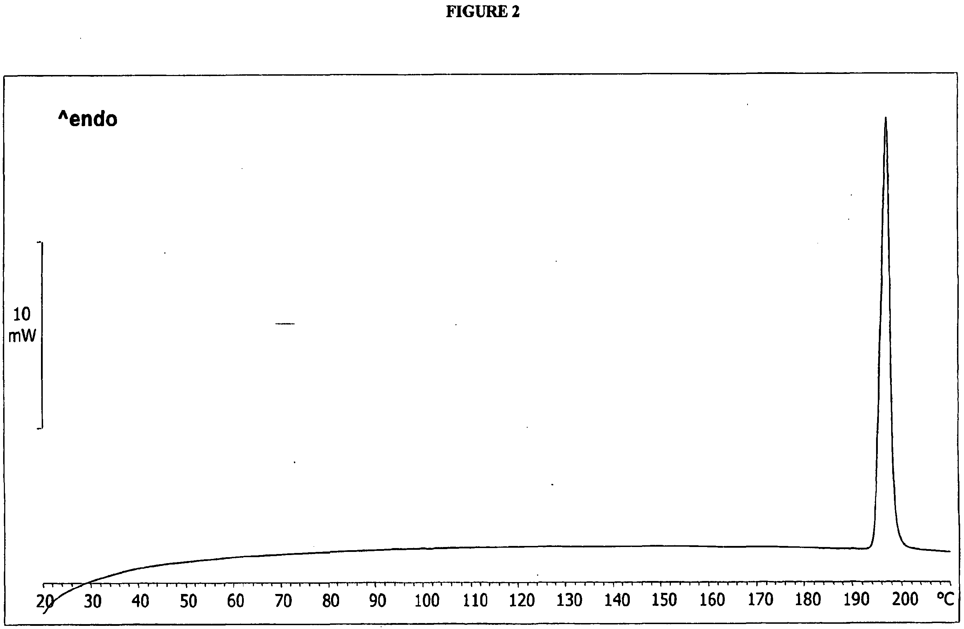

Figure 2 is a DSC thermogram of toltrazuril prepared according to example 1

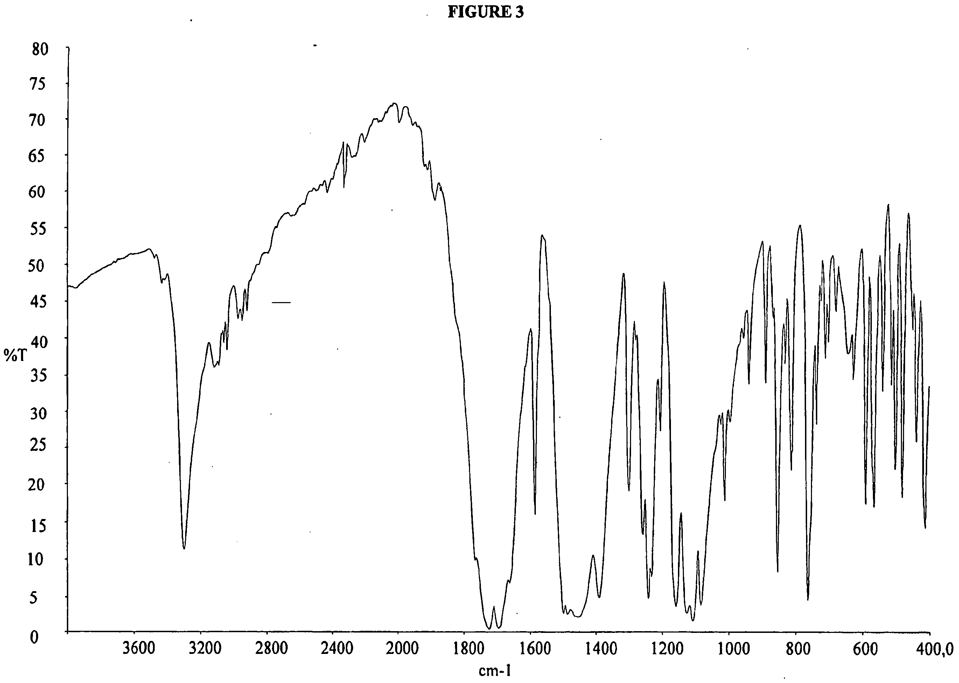

Figure 3 is a FT-IR spectrum of toltrazuril prepared according to example 1

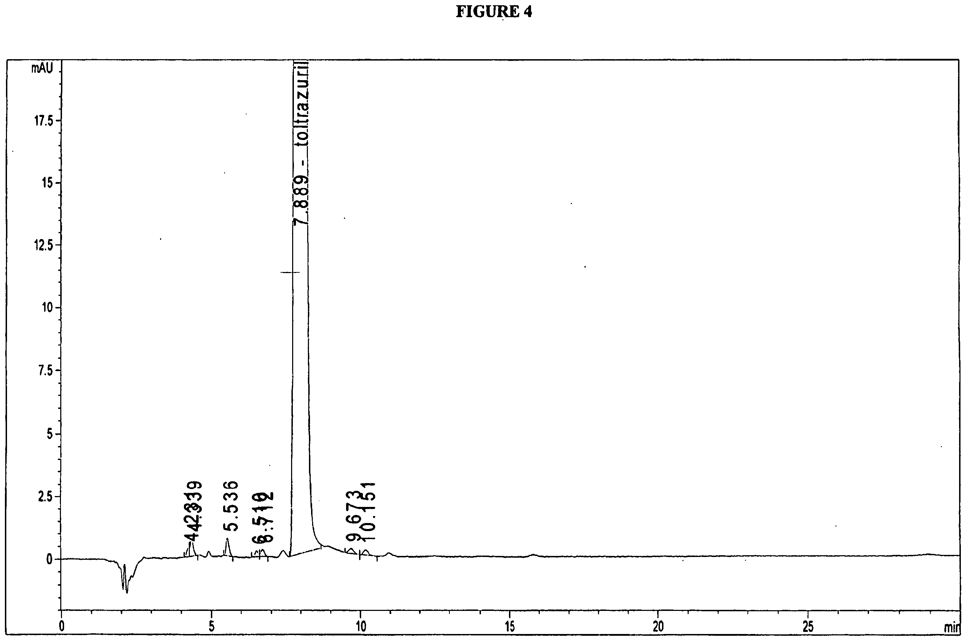

Figure 4 is a HPLC chromatogram of toltrazuril prepared according to example 1

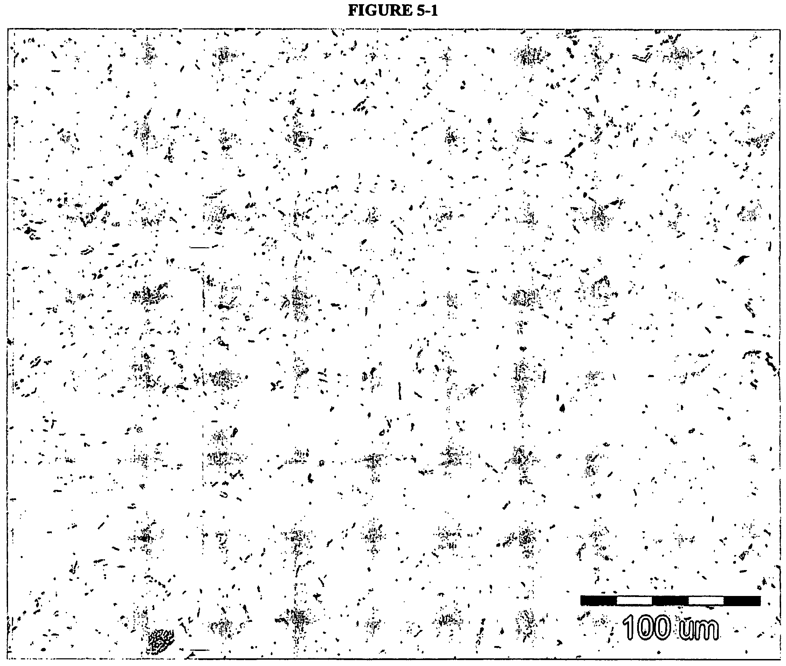

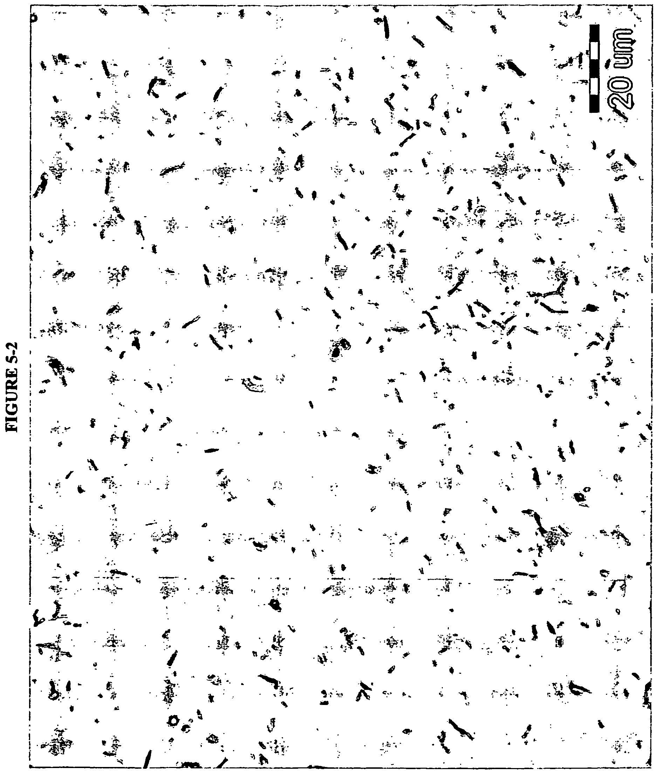

Figure 5 are pictures of particles of toltrazuril prepared according to example 1
Figure 6 is a dissolution profile of toltrazuril from toltrazuril suspension prepared according to example 8

X-ray powder diffraction patterns were obtained on a Phillips PW3040/60 X Pert PRO powder diffractometer with CuKα radiation of 1.541874A.

DSC scans were recorded on a DSC 822e Mettler Toledo scanning calorimeter. Samples of approximately 3 mg were scanned at a heating rate of 10°C/min under nitrogen atmosphere. FT-IR spectra (KBr discs) were recorded over a wave number range of 400-4000 cm⁻¹ on a Perkin Elmer Spectrum GX FT-IR spectrometer at a resolution of 4 cm⁻¹.

HPLC chromatogram of related substances of toltrazuril was obtained on a liquid chromatograph with UV detector. Chromatographic separation was achieved using Gemini C18, 150x4.6mm 3µm analytical column. Chromatographic conditions used were: sample concentration of 0.5mg/ml, detection at 210nm, flow of 0.7ml/min, volume of injection 5µL. An isocratic elution using mobile phase 0.02M Na₂HPO₄:CH₃CN = 36:64 (V/V)) pH 6.0 was employed.

Specific surface area was measured by gas sorption system based on nitrogen adsorption, using Brunauer, Emmett and Teller (BET) method. Corresponding amount of sample was outgased in a vacuum at room temperature. After degasification the tube was weighed with sample and the analysis was proceeded.

### Description of the dissolution method:

Ph Eur paddle method, rotation 50 RPM, Dissolution media 0.1M HCl containing 3.5% Tween 20, Volume 900 ml, HPLC evaluation of dissolution samples was performed with Gemini C18 110Å, 50 x 4.6 mm i.d., 5 µm analytical column. Chromatographic conditions used were: detection at 244 nm, mobile phase 0.02 M NaH₂PO₄:CH₃CN = 36: 64 (V/V), flow of 0.8 ml/min, injection volume of 5 µl.

### DETAILED DESCRIPTION OF THE INVENTION

The inventors of the present invention have found a simple and economic method for preparing toltrazuril particles which have improved dissolution properties.
The first aspect of the present invention is to provide toltrazuril particles with specific surface area between 4 and 40 m²/g, preferably between 7 and 30 m²/g, more preferably between 10 and 25 m²/g and most preferably between 12 and 20 m²/g. Toltrazuril can be in an amorphous or in crystalline state, preferably in crystal form. Crystalline toltrazuril prepared according to present invention is characterized by an X-ray powder diffraction pattern having peaks at about 5.5, 14.1, 16.5, 18.6, 22.1 ± 0.2 degrees two-theta. Crystalline toltrazuril can be further characterized by an X-ray powder diffraction pattern having peaks at about 5.5, 10.9, 12.0, 14.1, 16.5, 18.6, 22.1 ± 0.2 degrees two-theta.

In a further aspect, the present invention provides a process for the preparation of toltrazuril particles according to the present invention comprising the steps of:
a) suspending toltrazuril in a solvent;
b) dissolving toltrazuril with an addition of a basifying agent to the suspension;
c) precipitation of toltrazuril with an addition of an acidifying agent to the solution or with an addition of toltrazuril solution to an acidifying agent;
d) separation of precipitated toltrazuril from a liquid phase; and
e) optional further maceration of toltrazuril.

Solvent according to present invention can be any solvent such as solvent selected from the group consisting of water, alcohols, ketones, nitriles, sulfoxides, amides, ethers, esters and/or any mixtures thereof. Preferably, the solvent is water or aqueous solvent mixture. More preferably, the solvent is water.
The term "basifying agent" means an agent able to increase the pH of toltrazuril suspension. For example, suitable basifying agents according to present invention can be selected from the group consisting of basic hydroxides, alkoxides or basic ammonium compounds. Preferably, the basifying agent is selected from the group consisting of alkali or earth-alkali metal hydroxides or alkoxides, ammonia and/or alkyl amines. More preferably, the basifying agent is sodium hydroxide, potassium hydroxide or ammonia. Most preferably potassium or sodium hydroxide is used.
pH of suspension while dissolving toltrazuril can vary and is dependent on the selection of a solvent. In the case of water or aqueous solvent mixture as a solvent, the pH is preferably above 10, more preferably above 11, most preferably between 11 and 13, in particular between 11.5 and 13.
The term "acidifying agent" means an agent able to lower the pH of toltrazuril solution. For example, suitable acidifying agents according to present invention can be selected from the group consisting of hydrohalogenic acids, carboxylic acids, nitric acid, sulfuric acid, phosphoric acid and/or sulfonic acids. Preferably, the acidifying agent is hydrohalogenic acid, more preferably the acidifying agent is hydrochloric acid or hydrobromic acid.
Separation of precipitated toltrazuril according to present invention can be achieved by any know separation technique such as filtration.

In case of water as the main solvent, organic solvent can be optionally added to the basic solution before toltrazuril precipitation. The addition of organic solvent gives better filterability of precipitated toltrazuril. The organic solvent can be selected from the group consisting of alcohols such as n-butanol, ketones, nitriles and/or esters.
The precipitation can be performed at any suitable temperature, preferably the temperature is in the range of 15-50°C, most preferably 20-25°C.
The concentration of toltrazuril in the basic solution is preferably in the range of 2-15% (w/w), preferably 2-5%. During precipitation the solution should be stirred very intensively. The obtained toltrazuril after filtration could be further purified with maceration, i.e. by stirring precipitated toltrazuril in a solvent. Maceration could be performed in any solvent such as for example water, alcohols, ketones, nitriles, sulfoxides, amides, ethers, esters and/or any mixtures thereof. Preferably, the solvent for maceration is water or aqueous solvent mixture. In the case of water as maceration solvent the ratio water to toltrazuril is preferably in the range of 5-30 (w/w).
Toltrazuril can be dried by conventional methods, preferably by air or vacuum drying.
If toltrazuril particles according to present invention show agglomeration, the agglomerates can be broken by common methods, such as sieving, ultrasound or milling. Milling can be achieved using, for example, a crusher, pulverizer, grinding mill, attrition mill, ball mill, sand mill, bead mill, chaser mill, jar mill, hammer mill, impact grinding mill, homogenizer (such as APV homogenizer), air jet mill, colloid mill, vibratory mill, ultrasound mill, ultrasonic mill and/or micronizer. Depending on the type of mill used, milling can be wet and/or dry.

Further aspect of the invention is use of toltrazuril particles prepared according to present invention for the preparation of toltrazuril formulations, such as solutions and especially suspensions. Still another aspect are pharmaceutical compositions comprising toltrazuril particles according to the present invention.

With a process according to present invention, toltrazuril can be prepared in a purity of more than 98%, preferably more than 99.0%, most preferably more than 99.5 %. The purity is determined by HPLC.

Toltrazuril used in the present invention can be prepared by known methods in prior art such as by methods disclosed in GB 1 461 375, US 4,219,552, DE 35 16 631, EP 0 201 030, DE 42 39 000, CN1896068, CN101108831, CN1927846 and/or CN101265236.

The invention is illustrated by reference to the following examples. However, the examples are not intended to limit any scope of the claim anyway. It will be apparent to those skilled in the art that many modifications, both to materials and methods, may be practiced without departing from the purpose and interest of this invention.

### EXAMPLES

### Example 1

5 g of toltrazuril was suspended in 100 ml of demineralized water. During intensive stirring pH correction with 2M aqueous potassium hydroxide was followed until whole substance was dissolved (pH is above 11.5). Then precipitation of toltrazuril with 6M hydrochloric acid was performed. In the first step the pH was corrected to 1.5 and then pH was corrected to 7.7. The product was filtered off and washed with water. Wet toltrazuril after filtration was suspended in 40 ml of water and stirred. After one hour of maceration by intensive stirring the product was filtered off, washed with water and vacuum dried at 50°C.
Specific surface area: 13.8 ₘ²/g

Toltrazuril particles were further deagglomerated on air jet mill. The obtained micronized toltrazuril had specific surface area of 14.1 m²/g.

### Example 2

5 g of toltrazuril was suspended in 100 ml of demineralized water. During intensive stirring pH correction with 2M aqueous potassium hydroxide was followed until whole substance was dissolved (pH was above 11.5). Then precipitation of toltrazuril with 6M hydrochloric acid was performed at 50 °C. The pH range of final precipitation was 7.3 to 7.9. The product was filtered off and washed with water. Wet product was vacuum dried at 50°C.
Specific surface area: 15 m²/g

### Example 3

5 g of toltrazuril was suspended in 100 ml of demineralized water. During intensive stirring pH correction with 2M aqueous potassium hydroxide was followed until whole substance was dissolved (pH was above 11.5). Then precipitation of toltrazuril with 6M hydrochloric acid was performed. The pH range of final precipitation was 7.3 to 7.9. The product was filtered off and washed with water. Wet product was vacuum dried at 50°C.
Specific surface area: 13.8 m²/g

### Example 4

5 g of toltrazuril was suspended in 50 ml of demineralized water. During intensive stirring pH correction with 2M aqueous potassium hydroxide was followed until whole substance was dissolved (pH was above 11.5). In another vessel water (150 ml) acidified with hydrochloric acid at about pH 1.5 was prepared. During intensive stirring the solution of toltrazuril was continuously added and pH was corrected with HCl acid in the range 1-3. Obtained suspension was stirred for another one hour and pH was corrected to 7.7. The product was filtered off and vacuum dried at 50°C.
Specific surface area: 15.2 m²/g

### Example 5

10 g of toltrazuril was suspended in 50 ml of demineralized water. During intensive stirring pH correction with 2M aqueous potassium hydroxide was followed until whole substance was dissolved at room temperature (pH was above 11.5). In another vessel water (90 ml) acidified with hydrochloric acid at about pH 2 was prepared. During intensive stirring the solution of toltrazuril was continuously added into prepared acidified water with a pH 1.5-3.0 at 20 °C (in about 1 hour) and pH was corrected with HCl acid in the range 1-3. Obtained suspension was stirred for another one hour and pH was corrected to 7.7. The product was filtered off and resuspended in 120 mL of water, stirred for about one hour, filtered off and vacuum dried at 50°C.
Specific surface area: 19.5 m2/g

### Example 6

5 g of toltrazuril was suspended in 100 ml of demineralized water. During intensive stirring pH correction with 2M potassium hydroxide was followed until whole substance was dissolved (pH was above 11.5). 4 ml of n-butanol was added and the precipitation of toltrazuril with 6M hydrochloric acid was performed. The pH range of final precipitation was 7.3 to 7.9. The product was filtered off and washed with water. Wet product was vacuum dried at 50°C.
Specific surface area: 18.8 m²/g

**Example 7: Toltrazuril oral solution 2.5%**

| | Amount [g] |
|---|---|
| Toltrazuril | 2.50 |
| Triethanolamine | 30.00 |
| Polyethylene glycol | 80.70 |

Triethanolamine and polyethylene glycol are stirred and heated to around 60°C in a suitable stirrer. Toltrazuril, prepared according to any process as disclosed in examples 1-6, was added into solution and stirred, while heating, until a clear solution was produced. A solution was filtered and dispensed in suitable containers.

**Example 8: Toltrazuril oral suspension 5%**

| | Amount [g] |
|---|---|
| Toltrazuril | 5.000 |
| Propylene glycol | 10.000 |
| Sodium docusate | 0.250 |
| Simethicone emulsion | 0.050 |
| Bentonite | 0.350 |
| Citric acid hydrate | 0.500* |
| Xanthan gum | 0.250 |
| Sodium propionate | 0.210 |
| Sodium benzoate | 0.210 |
| Purified water | ad 100.000 ml |

| | |
|---|---|
| *The amount of citric acid can vary in order to adjust pH from 3.5 to 4.5. | |

Propylene glycol, simethicone emulsion, sodium propionate, sodium benzoate and purified water are stirred together in a suitable stirrer. Toltrazuril, prepared according to any process as disclosed in examples 1-6, was added and mixed until a homogeneous suspension was obtained. Xanthan gum was separately dissolved in water and added into a suspension. Bentonite and sodium docusate were also prepared separately in aqueous suspension, heated and after cooling added into a suspension. At the end aqueous solution of citric acid was added in order to adjust pH. A homogeneous suspension was filtered and dispensed in suitable containers.

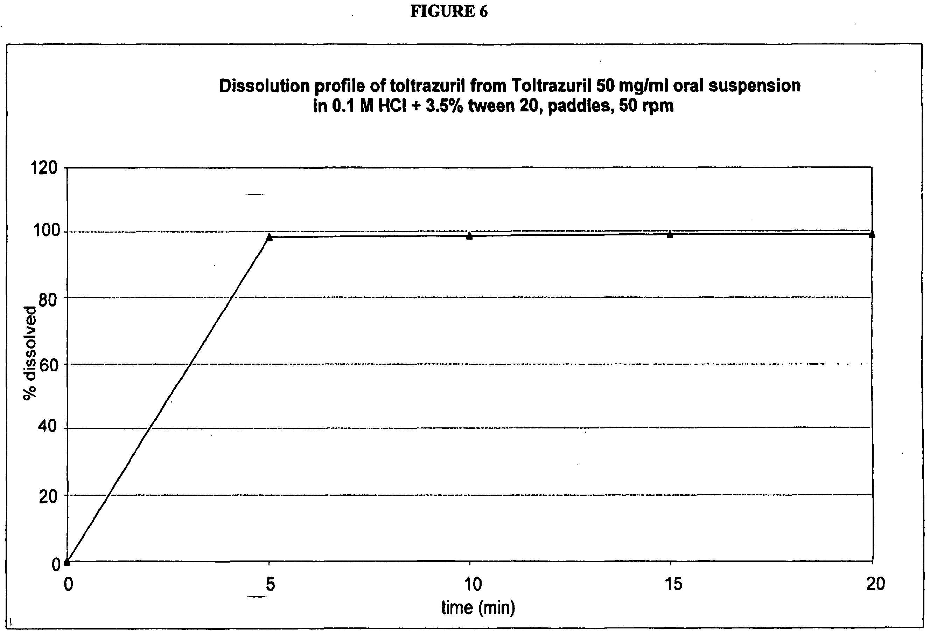

Figure 6 shows the dissolution profile of toltrazuril prepared according to present invention.

**Example 9: Toltrazuril oral suspension 5%**

| | Amount [g] |
|---|---|
| Toltrazuril | 5.00 |
| Propylene glycol | 10.00 |
| Sodium docusate | 0.25 |
| Simethicone emulsion | 0.05 |
| Bentonite | 0.35 |
| Citric acid hydrate | 0.50* |
| Xanthan gum | 0.25 |
| Sodium propionate | 0.21 |
| Sodium benzoate | 0.21 |
| Purified water | ad 100 ml |

| | |
|---|---|
| *The amount of citric acid can vary in order to adjust pH from 3.5 to 4.5. | |

Bentonite was dispersed in purified water, heated and after swelling the obtained dispersion was cooled to temperature of about 40°C. Sodium propionate and sodium benzoate were dissolved in propylene glycol and xanthan gum was added and mixed. The obtained dispersion was added to bentonite dispersion and mixed. Toltrazuril, prepared according to any process as disclosed in examples 1-6, simethicone emulsion and sodium docusate were separately dispersed in purified water and homogenized to obtain 20% concentrated suspension of toltrazuril. The concentrate of toltrazuril was admixed to dispersion above and mixed until a homogeneous suspension was obtained. At the end aqueous solution of citric acid was added in order to adjust pH. Finally, suspension was filtered and dispensed in suitable containers.

Toltrazuril particles and formulations prepared according to examples were put on stability test at 50°C/75RH, 28 days. The test did not show any increase of impurities.

## Claims

1. Toltrazuril particles having a specific surface area between 4 and 40 m²/g.

2. Toltrazuril particles according to claim 1, having a specific surface area between 7 and 30 m²/g.

3. Toltrazuril particles according to claim 2, having a specific surface area between 10 and 25 m²/g.

4. Toltrazuril particles according to claim 1,2 or 3, being in a crystalline state.

5. Toltrazuril particles according to claim 4, **characterized by** an X-ray powder diffraction pattern having peaks at about 5.5, 14.1, 16.5, 18.6, 22.1 ± 0.2 degrees two-theta.

6. A process for the preparation of toltrazuril particles according to claim 1, 2 or 3, comprising the steps of:
a) suspending toltrazuril in a solvent;
b) dissolving toltrazuril with an addition of a basifying agent to the suspension;
c) precipitation of toltrazuril with an addition of an acidifying agent to the solution or with an addition of toltrazuril solution to an acidifying agent;
d) separation of precipitated toltrazuril from a liquid phase; and
e) optional further maceration of toltrazuril.

7. The process according to claim 6, wherein the solvent is selected from the group consisting of water, alcohols, ketones, nitriles, sulfoxides, amides, ethers, esters and/or any mixtures thereof.

8. The process according to claim 7, wherein the solvent is water or aqueous solvent mixture.

9. The process according to claim 6, wherein pH of the suspension while dissolving toltrazuril is maintained above 10.

10. The process according to claim 9, wherein pH of the suspension while dissolving toltrazuril is maintained between 11 and 13.

11. The process according to claim 6, wherein the basifying agent is selected from the group consisting of basic hydroxides, alkoxides and/or basic ammonium compounds.

12. The process according to claim 11, wherein the basifying agent is selected from the group consisting of alkali or earth-alkali metal hydroxides or alkoxides, ammonia and/or alkyl amines.

13. The process according to claim 12, wherein the basifying agent is potassium or sodium hydroxide.

14. The process according to claim 6, wherein the acidifying agent is selected from the group consisting of hydrohalogenic acids, carboxylic acids, nitric acid, sulfuric acid, phosphoric acid and/or sulfonic acids.

15. The process according to claim 14, wherein the acidifying agent is hydrochloric acid or hydrobromic acid.

## Patentansprüche

1. Toltrazurilpartikel, die eine spezifische Oberfläche zwischen 4 und 40 m²/g aufweisen.

2. Toltrazurilpartikel nach Anspruch 1, die eine spezifische Oberfläche zwischen 7 und 30 m²/g aufweisen.

3. Toltrazurilpartikel nach Anspruch 2, die eine spezifische Oberfläche zwischen 10 und 25 m²/g aufweisen.

4. Toltrazurilpartikel nach Anspruch 1, 2 oder 3, die in kristallinem Zustand vorliegen.

5. Toltrazurilpartikel nach Anspruch 4, die durch ein Pulver-Röntgenbeugungsmuster gekennzeichnet sind, das Signale bei ungefähr 5,5, 14,1, 16,5, 18,6, 22,1 ± 0,2 Grad 2θ aufweist.

6. Verfahren zur Darstellung von Toltrazurilpartikeln nach Anspruch 1, 2 oder 3, bei dem
(a) man Toltrazuril in einem Lösungsmittel suspendiert,
(b) man Toltrazuril durch Zugabe von Basifizierungsmittel zu der Suspension in Lösung bringt,
(c) man Toltrazuril durch Zugabe von Säuerungsmittel zu der Lösung oder durch Zugabe von Toltrazurillösung zu einem Säuerungsmittel ausfällt,
(d) man ausgefallenes Toltrazuril von der flüssigen Phase trennt, und
(e) man Toltrazuril wahlweise weiter mazeriert.

7. Verfahren nach Anspruch 6, bei dem das Lösungsmittel aus der Gruppe ausgewählt ist, die aus Wasser, Alkoholen, Ketonen, Nitrilen, Sulfoxiden, Amiden, Ethern, Estern und/oder beliebigen Mischungen davon besteht.

8. Verfahren nach Anspruch 7, bei dem das Lösungsmittel Wasser oder eine wässrige Lösungsmittelmischung ist.

9. Verfahren nach Anspruch 6, bei dem ein pH-Wert der Suspension während des Lösens von Toltrazuril von über 10 aufrechterhalten wird.

10. Verfahren nach Anspruch 9, bei dem ein pH-Wert der Suspension während des Lösens von Toltrazuril zwischen 11 und 13 aufrechterhalten wird.

11. Verfahren nach Anspruch 6, bei dem das Basifizierungsmittel aus der Gruppe ausgewählt ist, die aus basischen Hydroxiden, Alkoholaten und/oder basischen Ammoniumverbindungen besteht.

12. Verfahren nach Anspruch 11, bei dem das Basifizierungsmittel aus der Gruppe ausgewählt ist, die aus Alkali- oder Erdalkalimetallhydroxiden oder -alkoholaten, Ammoniak und/oder Alkylaminen besteht.

13. Verfahren nach Anspruch 12, bei dem das Basifizierungsmittel Kalium- oder Natriumhydroxid ist.

14. Verfahren nach Anspruch 6, bei dem das Säuerungsmittel aus der Gruppe ausgewählt ist, die aus Halogenwasserstoffsäuren, Carbonsäuren, Salpetersäure, Schwefelsäure, Phosphorsäure und/oder Sulfonsäuren besteht.

15. Verfahren nach Anspruch 14, bei dem das Säuerungsmittel Salzsäure oder Bromwasserstoffsäure ist.

## Revendications

1. Particules de toltrazuril qui présentent une aire spécifique valant de 4 à 40 m²/g.

2. Particules de toltrazuril conformes à la revendication 1, qui présentent une aire spécifique valant de 7 à 30 m²/g.

3. Particules de toltrazuril conformes à la revendication 2, qui présentent une aire spécifique valant de 10 à 25 m²/g.

4. Particules de toltrazuril conformes à la revendication 1, 2 ou 3, qui se présentent dans un état cristallin.

5. Particules de toltrazuril conformes à la revendication 4, qui se caractérisent par un spectre de diffraction des rayons X, à l'état de poudre, qui présente des pics aux alentours des valeurs suivantes de 2θ, à 0,2° près : 5,5°, 14,1°, 16,5°, 18,6° et 22,1°.

6. Procédé de préparation de particules de toltrazuril conformes à la revendication 1, 2 ou 3, comportant les étapes suivantes :
a) mettre du toltrazuril en suspension dans un solvant ;
b) dissoudre le toltrazuril en ajoutant à la suspension un agent basifiant ;
c) faire précipiter le toltrazuril, soit en ajoutant à la solution un agent acidifiant, soit en ajoutant la solution de toltrazuril à un agent acidifiant ;
d) séparer le précipité de toltrazuril d'avec la phase liquide ;
e) et en option, faire en outre macérer le toltrazuril.

7. Procédé conforme à la revendication 6, dans lequel le solvant est choisi dans l'ensemble constitué par l'eau, les alcools, cétones, nitriles, sulfoxydes, amides, éthers et esters, et/ou n'importe quels mélanges de tels solvants.

8. Procédé conforme à la revendication 7, dans lequel le solvant est de l'eau ou un mélange d'eau et de solvant.

9. Procédé conforme à la revendication 6, dans lequel, pendant la dissolution du toltrazuril, on maintient le pH de la suspension à une valeur supérieure à 10.

10. Procédé conforme à la revendication 9, dans lequel, pendant la dissolution du toltrazuril, on maintient le pH de la suspension à une valeur située dans l'intervalle allant de 11 à 13.

11. Procédé conforme à la revendication 6, dans lequel l'agent basifiant est choisi dans l'ensemble constitué par les hydroxydes basiques, les alcoolates et/ou les composés d'ammonium basiques.

12. Procédé conforme à la revendication 11, dans lequel l'agent basifiant est choisi dans l'ensemble constitué par les hydroxydes et alcoolates de métal alcalin ou alcalino-terreux, l'ammoniac et/ou les alkyl-amines.

13. Procédé conforme à la revendication 12, dans lequel l'agent basifiant est de l'hydroxyde de potassium ou de sodium.

14. Procédé conforme à la revendication 6, dans lequel l'agent acidifiant est choisi dans l'ensemble constitué par les acides halogénohydriques, les acides carboxyliques, l'acide nitrique, l'acide sulfurique, l'acide phosphorique et/ou les acides sulfoniques.

15. Procédé conforme à la revendication 14, dans lequel l'agent acidifiant est de l'acide chlorhydrique ou de l'acide bromhydrique.
